# EUROPEAN PATENT APPLICATION

(11) **EP 2 754 734 A1**
(43) Date of publication of application: **16.07.2014**
(21) Application number: 12829235.6
(22) Date of filing: 07.09.2012
(51) Int. Cl.: C25D 11/04, C01G 3/04, C01G 5/02, C01G 23/047, C25D 11/18

(54) **ANTI-VIRUS ALUMINUM MEMBER AND METHOD FOR PRODUCING SAME**

(30) Priority: 07.09.2011 JP 2011195123
(71) Applicant: NBC Meshtec, Inc., Hino-shi, Tokyo 191-0053 (JP)
(72) Inventor: FUKUI, Yoko, Tokyo 191-0053 (JP); NAKAYAMA, Tsuruo, Tokyo 191-0053 (JP); FUJIMORI, Yoshie, Tokyo 191-0053 (JP)
(74) Representative: Vossius & Partner
(86) International application number: PCT/JP2012/005695
(87) International publication number: WO 2013/035343

(57) **Abstract**

[Problem] To provide an anti-virus aluminum member capable of minimizing secondary infection by deactivating viruses in a short period of time even when viruses adhere thereto, regardless of whether a viral envelope is present, and useful for application in door knobs, handrails, air-conditioner fins or the like.

[Solution] An anti-virus aluminum member capable of deactivating viruses that adhere thereto is characterized in that an anti-virus inorganic compound is present in the pores of an anodized membrane provided with multiple pores and obtained by anodizing aluminum or an aluminum alloy.

## Description

### Technical Field

The present invention relates to an anti-virus aluminum member that adsorbs a virus and inactivates it in a short period of time, wherein the anti-virus aluminum member has a porous anodic oxide film formed by anodic oxidation.

### Background Art

In recent years, the deaths of people that are caused by SARS (severe acute respiratory syndrome) and viral infections such as norovirus and avian influenza have been reported. In particular, in 2009, the world was faced with a crisis of a "pandemic", which means a viral infection that spreads all over the world, due to the growth of transportation and a mutation of a virus. Furthermore, serious damage caused by a virus such as foot-and-mouth disease virus has also emerged. Therefore, urgent countermeasures are required. To address such a situation, the development of an anti-virus substance based on a vaccine is being hastened. However, a vaccine can only prevent infection with a specific virus because of its specificity. Furthermore, a norovirus, which is a type of virus that causes acute nonbacterial gastroenteritis, is known to cause food poisoning from shellfish such as oyster and also to cause an oral infection from infected individual's stool or vomit, or dust originating from dried stool or vomit. Norovirus contagion to patients and health care professionals occurs through an environment including a door knob, a handrail, a wall, or equipment such as an air-conditioner. Thus, a norovirus is also becoming a more serious social problem. Therefore, development of an anti-virus material that adsorbs a variety of viruses and can inactivate the adsorbed viruses efficiently is highly desirable.

Examples of anti-virus materials may include a virus-inactivating sheet that uses a complex that contains an inorganic porous crystal within a resin, in which the inorganic porous crystal supports an anti-virus metal ion such as a silver ion and a copper ion (Patent Literature 1), a virus-inactivating sheet in which inorganic fine particles with an anti-virus effect are supported on a substrate (Patent Literature 2), and the like.

### Citation List

### Patent Literature

Patent Literature 1: Japanese Patent Application Laid-Open No. 2010-30984
Patent Literature 2: WO 2011/040048

### Summary of Invention

### Technical Problem

However, although the method in which an inorganic porous crystal is contained within a resin is applicable to a fibrous fabric, the method is not applicable to door knobs, handrails, or fin materials for air-conditioners. Furthermore, although the method that uses inorganic fine particles with an anti-virus effect is excellent in both versatility and effectiveness, problems are caused by aggregation of the inorganic fine particles when a smaller particle size of the inorganic fine particles is used. The problems are, for example, reduced efficiency and peeling off due to reduced adhesion between the agglomerate and the substrate.

Viruses can be classified into viruses with no envelope such as a norovirus and viruses with an envelope such as an influenza virus. Even though a pharmaceutical agent can inactivate a virus with an envelope, the agent may not act on a virus with no envelope. Furthermore, in the case of door knobs, handrails, fin materials for air-conditioners, or the like, viruses adhering to an infected individual or droplets scattered by a cough float in the air and adhere to surfaces of the door knobs, the handrails, the finmaterials or the like. Lipid, protein, and the like that are contained in body fluids such as sweat and saliva may adhere to their surfaces. Therefore, it is preferable to be able to inactivate a virus even in an environment in which lipid, protein, and the like are present.

Therefore, it is an object of the present invention to provide an anti-virus aluminum member that can inactivate viruses in a short period of time when the viruses adhere to the member and inhibit a secondary infection regardless of whether a viral envelope is present to solve the above-mentioned problems. The inventive anti-virus aluminum member is useful for application to door knobs, handrails, wheelchairs, bed components, pipe chairs, window sashes, bicycle frames, interior decorative materials, fin materials for air-conditioners, and the like.

### Solution to Problem

Thus, a first aspect of the present invention provides an anti-virus aluminum member that can inactivate a virus adhering to the anti-virus aluminum member, wherein an anodic oxide film obtained by anodizing aluminum or an aluminum alloy has a large number of pores, and an anti-virus inorganic compound is present within the pores.

Furthermore, a second aspect of the present invention provides the anti-virus aluminum member of the above-mentioned first aspect of the present invention, wherein a surface film is formed on the surface of the anodic oxide film that has the above-mentioned anti-virus inorganic compound present within the above-mentioned pores, the surface film including an anti-virus inorganic compound and a binder resin.

Furthermore, a third aspect of the present invention provides the anti-virus aluminum member of the above-mentioned second aspect of the present invention, wherein the above-mentioned surface film further includes inorganic fine particles different from the above-mentioned anti-virus inorganic compound.

Furthermore, a fourth aspect of the present invention provides the anti-virus aluminum member of the third aspect of the present invention, wherein the inorganic fine particles included in the above-mentioned surface film are a photocatalytic substance.

Furthermore, a fifth aspect of the present invention provides the anti-virus aluminum member of the fourth aspect of the present invention, wherein the above-mentioned photocatalytic substance is a visible light-responsive photocatalytic substance.

Furthermore, a sixth aspect of the present invention provides the anti-virus aluminum member of any one of the third to fifth aspects of the present invention, wherein the surface of the inorganic fine particle included in the above-mentioned surface film is covered with a silane monomer.

Furthermore, a seventh aspect of the present invention provides the anti-virus aluminum member of any one of the above-mentioned second to sixth aspects of the present invention, wherein the above-mentioned binder resin is a silane compound.

Furthermore, an eighth aspect of the present invention provides the anti-virus aluminum member of any one of the first to seventh aspects of the present invention, wherein the above-mentioned anti-virus inorganic compound is at least one of a monovalent copper compound and an iodine compound.

Furthermore, a ninth aspect of the present invention provides the anti-virus aluminum member of the eighth aspect of the present invention, wherein the above-mentioned monovalent copper compound is at least one of a chloride, an acetic acid compound, a sulfide, an iodine compound, a bromide, aperoxide, anoxide, andathiocyanide.

Furthermore, a tenth aspect of the present invention provides an anti-virus aluminum member of the ninth aspect of the present invention, wherein the above-mentioned monovalent copper compound is at least one of CuCl, CuBr, Cu(CH₃COO), CuSCN, Cu₂S, Cu₂O, and CuI.

Furthermore, a eleventh aspect of the present invention provides the anti-virus aluminum member of any one of the eighth to tenth aspects of the present invention, wherein the above-mentioned iodine compound is at least one of CuI, AgI, SbI₃, IrI₄, GeI₄, GeI₂, SnI₂, SnI₄, TlI, PtI₂, PtI₄, PdI₂, BiI₃, AuI, AuI₃, FeI₂, CoI₂, NiI₂, ZnI₂, HgI, and InI₃.

Furthermore, a twelfth aspect of the present invention provides a method for producing an anti-virus aluminum member. The method includes the steps of : anodizing an aluminummaterial made of aluminum or an aluminum alloy to form pores on the surface of the aluminum material; and depositing an anti-virus inorganic compound within the above-mentioned pores of the above-mentioned aluminum material with the above-mentioned pores formed on the surface of the aluminum material by electrochemical treatment.

Furthermore, a thirteenth aspect of the present invention provides the method for producing an anti-virus aluminum member of the twelfth aspect of the present invention, wherein the step of depositing the anti-virus inorganic compound comprises:
depositing at least one of Cu and Ag within the above-mentioned pores by electrochemical treatment; immersing the aluminum material with at least one of Cu and Ag having been deposited within the above-mentioned pores in an iodine ion-containing electrolyte; and
depositing CuI or AgI, which is the anti-virus inorganic compound, within the above-mentioned pores by electrochemical treatment of the immersed aluminum material.

### Advantageous Effects of Invention

In accordance with the present invention, it is possible to provide an aluminum member with excellent durability that can maintain its anti-virus property for a long period of time, even when the aluminum member is used for door knobs, handrails, or fin materials for air-conditioners.

### Brief Description of Drawings

Fig. 1 is a sectional view of an anti-virus aluminum member of a first embodiment of the present invention.
Fig. 2 is a sectional view of an anti-virus aluminum member of a second embodiment of the present invention.
Fig. 3 is a sectional view of an anti-virus aluminum member of a third embodiment of the present invention.
Fig. 4 is a sectional view of an anti-virus aluminum member of a fourth embodiment of the present invention.

### Description of Embodiments

Hereinbelow, embodiments of the present invention will be described in detail with reference to the drawings.

### (First Embodiment)

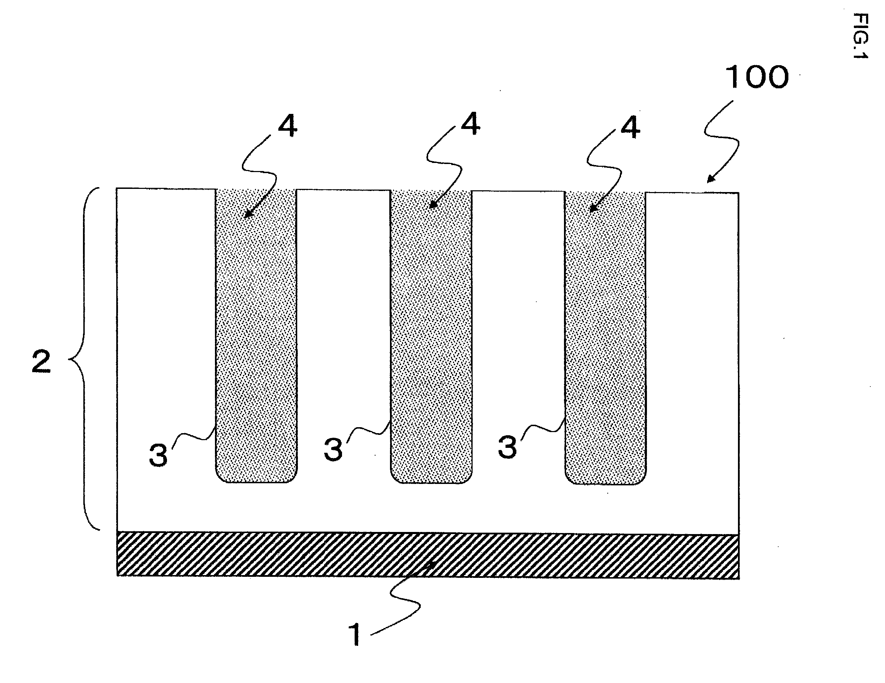

Fig. 1 is an enlarged schematic view of part of the cross section of an anti-virus aluminum member 100 of the first embodiment of the present invention. The aluminum member 100 has an anodic oxide film 2 that is formed on the surface part of the member by anodizing aluminum or an aluminum alloy. The anodic oxide film 2 is a so-called porous alumina that has a large number of pores 3 formed on its surface, the pores having openings. A metal layer 1 of original aluminum or an original aluminum alloy that is not anodized lies on the side near the bottom of the pores 3 (the opposite side to the surface with the openings of the aluminum member 100). In this embodiment of the present invention, as shown in Fig. 1, a deposit 4 including an anti-virus inorganic compound is deposited within the pores 3 of the anodic oxide film 2 to fill the pores 3. To facilitate understanding, Fig. 1 shows a view in which the pores 3 are completely filled with the deposit 4 that was deposited in the pore 3. However, the deposit 4 that was deposited in the pore 3 may be any amount as long as it is deposited at least on the bottom of the pore 3 or in part of the pore 3.

Aluminum and an aluminum alloy defined in accordance with JISH4000, a clad material obtained by laminating aluminum on a steel sheet, or a material having a thin aluminum film formed by a physical method such as ion plating or sputtering on the surface of a resin can be used as aluminum or an aluminum alloy. On the surface of such aluminum or an aluminum alloy, the anodic oxide film 2 having the pores 3 is formed by a known method for anodic oxidation treatment. The anodic oxide film 2 having the pores 3 is formed by using aluminum or an aluminum alloy as an anode and applying a direct current voltage or an alternating current voltage. This is carried out, for example in an aqueous solution containing an acid such as sulfuric acid, phosphoric acid, chromic acid, or oxalic acid, or in an aqueous solution in which a small amount of sulfuric acid is added to an aromatic sulfonic acid or an aliphatic sulfonic acid such as sulfosalicylic acid, sulfophthalic acid, sulfomaleic acid, or sulfoitaconic acid. Although the thickness of the anodic oxide film 2 having the pores 3 is not particularly limited, the thickness is preferably approximately 1 µm to 50 µm.

The pores 3 of the anodic oxide film 2 of the present invention have a deposit 4 including an anti-virus inorganic compound deposited therein so as to be filled with the deposit 4. Preferably, the deposit 4 is at least one of a monovalent copper compound and an iodine compound.

Examples of the monovalent copper compound may include Cu₂O, CuOH, Cu₂S, CuSCN, CuBr, Cu(CH₃COO), CuI, and the like. For example, the pores 3 of the anodic oxide film 2 are filled with Cu₂O or CuOH in the following manner. That is, the aluminum member on which the anodic oxide film 2 is formed is immersed in a copper ion-containing aqueous solution. Then, a platinum electrode, a carbon electrode, or the like is used as a counter electrode and an alternating current voltage or a direct current voltage is applied thereto. In this manner, Cu₂O or CuOH can be deposited electrochemically wi thin the pores 3 so as to fill the pores 3.

As another example, the pores 3 of the anodic oxide film 2 are filled with a monovalent copper compound such as Cu₂S, CuSCN, CuBr, and CuI in the following manner. That is, first, the aluminum member on which the anodic oxide film 2 having the pores 3 is formed is immersed in an aqueous solution in which the fine particles of these copper compounds are suspended. Then, a platinum electrode, a carbon electrode, or the like is used as a counter electrode and an alternating current voltage or a direct current voltage is applied thereto. In this manner, the pores 3 of the anodic oxide film 2 can be filled with the intended compound by electrophoresis. In this case, the average particle diameter of the fine particles of the monovalent copper compound is preferably no more than approximately one-fifth of the diameter of the pore 3 in the anodic oxide film 2. In the present specification, an average particle diameter represents a volume-average particle diameter.

Examples of such an iodine compound may include CuI, AgI, SbI₃, IrI₄, GeI₄, GeI₂, SnI₂, SnI₄, TlI, PtI₂, PtI₄, PdI₂, BiI₃, AuI, AuI₃, FeI₂, CoI₂, NiI₂, ZnI₂, HgI, and InI₃. A method for depositing these compounds within the pores 3 of the anodic oxide film 2 is performed as follows. The aluminum member on which the anodic oxide film 2 having the pores 3 is formed is immersed in a dispersion of nanoparticles of these iodine compounds, and then, a platinum electrode, a carbon electrode, or the like is used as a counter electrode and an alternating current voltage or a direct current voltage is applied thereto to perform electrophoresis, thereby filling the pores 3 with the compound.

Another example for depositing an iodine compound on the aluminum member on which the anodic oxide film 2 having the pores 3 is formed will be described by using AgI. First, Ag is deposited within the pores 3 of the anodic oxide film 2 chemically and electrochemically, and then, a platinum electrode, a carbon electrode, or the like is used as a counter electrode and a direct current voltage is applied thereto in an iodine ion-containing solution. As a result, Ag deposited within the pores 3 of the anodic oxide film 2 and an iodine ion react to synthesize AgI within the pores 3 of the anodic oxide film 2. Finally, the anodic oxide film 2 with its pores 3 filled with AgI can be obtained.

Still another example will be described by using CuI. First, Cu₂O, CuOH, or the like including metal copper is deposited within the pores 3 of the anodic oxide film 2 on the aluminum member by electrochemical treatment. Then, the aluminum member is immersed in an iodine ion-containing aqueous solution. Then, a platinum electrode, a carbon electrode, or the like is used as a counter electrode, and a direct current voltage is applied between the aluminum member and the counter electrode. As a result, some of deposited metal copper, Cu₂O, CuOH, and the like react with an iodine ion to synthesize CuI, which can fill the pores 3 of the anodic oxide film 2. Other iodine compounds can also be deposited by using a similar method.

According to the first embodiment described above, the aluminum member 100 can quickly inactivate a virus that has adhered to it, because an anti-virus deposit 4 is deposited within the pores 3 to fill the pores 3. Furthermore, the deposit 4 is hardly soluble in water, and as a result of deposition it is bound to and adheres tightly within the pores 3 of the anodic oxide film 2 physically or mechanically. Therefore, the deposit 4 does not come off from the pore 3 and maintains the state of being anchored securely within the pore 3 of the anodic oxide film 2 for a long period of time, even if a special treatment for anchoring the anti-virus component is not performed. Therefore, according to this embodiment, an aluminum member that can exert an anti-virus effect stably for a long period of time can be provided.

It is preferable that an electrical potential control agent that can control the surface potential (a negative charge) to a positive charge exist on the surface on the side of the anodic oxide film 2 of the aluminum member 100 of this embodiment. The reason is as follows. A virus has a negative surface potential regardless of the type of its genome or whether a viral envelope is present. When the electrical potential control agent that controls the potential to a positive charge exists on the surface on the side of the anodic oxide film 2 of the aluminum member 100, the surface having the anti-virus deposit 4 exposed thereon, the surface potential becomes positive in contrast to a virus. Consequently, the aluminum member 100 can attract the virus. When a virus is attracted to the side of the anodic oxide film 2 successfully, the virus comes into contact with the anti-virus deposit 4 more easily, and therefore, an enhanced anti-virus effect can be obtained.

Such an electrical potential control agent is not particularly limited as long as it can control the surface potential of the aluminum member 100 to a positive charge. For example, a nonionic, an anionic, or a cationic surface active agent is preferable. Among these, a cationic surface active agent is particularly preferable.

### (Second Embodiment)

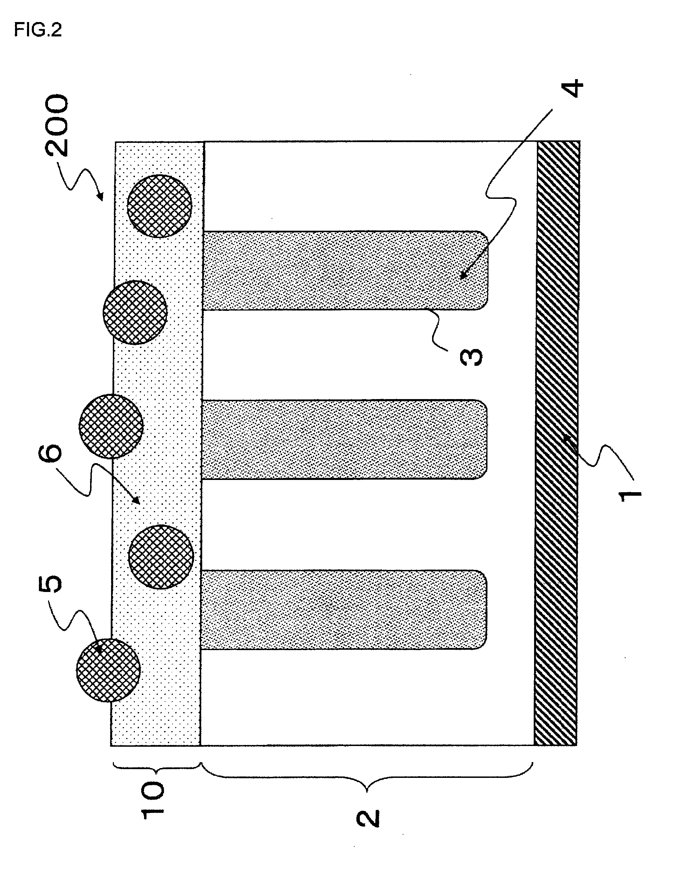

Next, an anti-virus aluminum member 200 of the second embodiment of the present invention will be described in detail with reference to Fig. 2.

Fig. 2 is an enlarged schematic view of part of the cross section of the anti-virus aluminum member 200 of the second embodiment of the present invention. As with the first embodiment, an anodic oxide film 2 having pores 3 formed by anodic oxidation is formed on the surface of a metal layer 1 of aluminum or its alloy, and a deposit 4 including an anti-virus inorganic compound is deposited within the pores 3 to fill the pores 3. Furthermore, a surface film 10 composed of inorganic fine particles 5 composed of an anti-virus inorganic compound and a resin binder 6 is formed on the surface of the anodic oxide film 2.

A known binder may be used as the resin binder 6. Specific examples of the resin binder may include a polyester resin, an amino resin, an epoxy resin, a polyurethane resin, an acrylic resin, a water soluble resin, a vinyl resin, a fluoro resin, a silicone resin, a cellulosic resin, a phenol resin, a xylene resin, a toluene resin, and a natural resin, for example, a drying oil such as castor oil, linseed oil, and tung oil.

In the resin binder 6, the inorganic fine particles 5 composed of the anti-virus inorganic compound are dispersed. At least one of a monovalent copper compound and an iodine compound may be used as the inorganic fine particles 5.

Examples of the monovalent copper compound used as the inorganic fine particles 5 may include a chloride, an acetic acid compound, a sulfide, an iodide, a bromide, a peroxide, an oxide, and a thiocyanide, and a monovalent iodine compound. For example, CuCl, Cu(CH₃COO), Cu₂S, CuI, CuBr, Cu₂O, and CuSCN may be used as a chloride, an acetic acid compound, a sulfide, an iodide, a bromide, a peroxide, an oxide, and a thiocyanide.

Examples of the iodine compound used as the inorganic fine particles 5 may include CuI, AgI, SbI₃, IrI₄, GeI₄, GeI₂, SnI₂, SnI₄, TlI, PtI₂, PtI₄, PdI₂, BiI₃, AuI, AuI₃, FeI₂, CoI₂, NiI₂, ZnI₂, HgI, and InI₃.

The particle diameter of the inorganic fine particles 5 composed of these anti-virus inorganic compounds is preferably 1 nm or more and 5 µm or less. An anti-virus effect becomes unstable over time at a particle diameter of less than 1 nm, while the strength of the film is reduced due to decreased retention by the resin binder 6 at a particle diameter of more than 5 µm. Thus, these particle diameters are not preferable.

Furthermore, the inorganic fine particles 5 are dispersed in the surface film 10 composed of the resin binder 6, preferably in an amount of 0.1% by mass or more and 80% by mass or less, and more preferably, in an amount of 0.1% by mass or more and 60.0% by mass or less. When the amount of the inorganic fine particles 5 is less than 0.1% by mass, the virus-inactivating effect is reduced compared to the effect when the amount falls within the above-mentioned range. Furthermore, even if the amount of the inorganic fine particles 5 is increased to more than 80.0% by mass, the virus-inactivating effect is virtually the same as the effect when the amount falls within the above-mentioned range. In addition, the binding property (retention effect) of the resin binder 6 is reduced, and therefore, the surface film 10 composed of the inorganic fine particles 5 and the resin binder 6 comes off more easily from the anodic oxide film 2 than when the amount falls within the above-mentioned range.

Furthermore, the surface film 10 of the second embodiment composed of the resin binder 6 and the inorganic fine particles 5 preferably includes a nonionic, an anionic, or a cationic surface active agent to increase the dispersibility of the inorganic fine particles 5. The surface active agent is not particularly limited as long as it can control the surface potential (a negative charge) of the surface film 10 to a positive charge when it is included in the resin binder 6. However, a cationic surface active agent is particularly preferable. The surface potential of a resin is generally negative. Furthermore, as described above, the surface potential of a virus is also negative regardless of the type of its genome or whether a viral envelope is present. Therefore, when a surface active agent is included in the surface film 10 along with the inorganic fine particles 5 composed of the anti-virus inorganic compound, the surface potential of the surface film 10 is controlled to a positive charge, and consequently a virus is adsorbed by the surface of the aluminum member 200 more easily. As a result, the anti-virus effect of the anti-virus inorganic fine particles 5 can be exerted more efficiently.

Furthermore, functional fine particles may be added to the surface film 10 of the second embodiment if necessary. Examples of the functional fine particle may include particles of other anti-virus compositions, an antibacterial composition, an antimold composition, an anti-allergen composition, a catalyst, an antireflective material, and a thermal barrier material.

A method for producing the aluminum member 200 of this embodiment will be described below. First, the anodic oxide film 2 that has a large number of pores 3 formed therein is formed on the surface of aluminum or an aluminum alloy by the method described in the first embodiment. Subsequently, the deposit 4 including an anti-virus inorganic compound is deposited within the pores 3 of the anodic oxide film 2. Then, the above-mentioned anti-virus inorganic fine particles 5 that were pulverized, for example, by a jet mill, the functional fine particles, and the like are mixed with any resin binder 6 to obtain a slurry. Then, the slurry is applied onto the surface of the aluminum member 200 and is allowed to dry. In this manner, the aluminum member 200 of this embodiment is produced.

According to the second embodiment described above, when the aluminum member 200 of this embodiment is used for a building material, an aluminum sash, or the like, the anti-virus property can be maintained over a long period of time. This long-lasting anti-virus property can be achieved because the deposit 4 deposited in the anodic oxide film 2 releases a monovalent copper ion, even when the anti-virus effect is reduced because of abrasion of the surface caused by certain usage environment.

### (Third Embodiment)

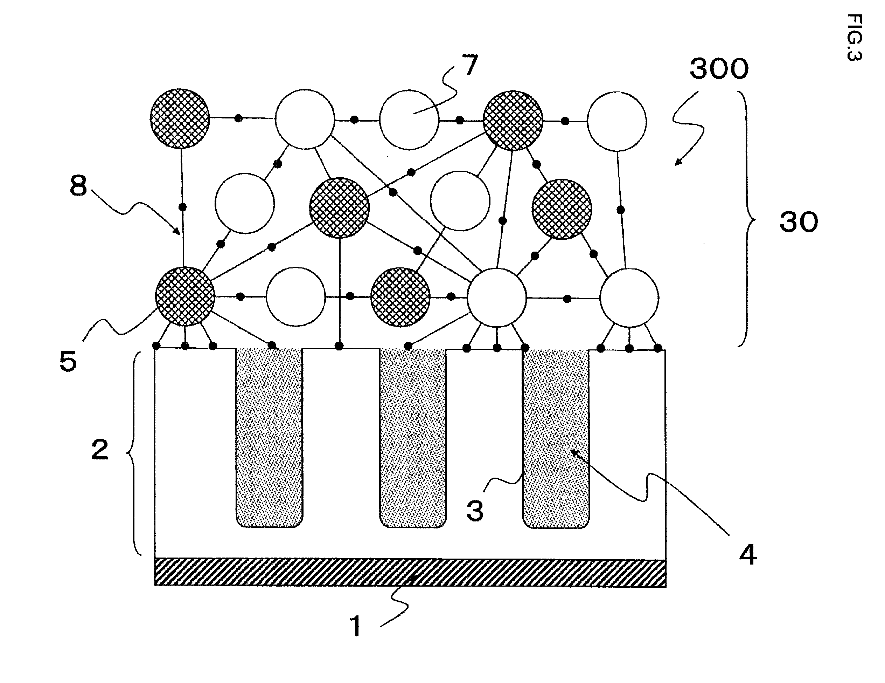

Next, an anti-virus aluminum member 300 of the third embodiment of the present invention will be described in detail with reference to Fig. 3.

Fig. 3 is an enlarged schematic view of part of the cross section of the anti-virus aluminum member 300 of the third embodiment of the present invention. In the third embodiment, a surface film 30 is formed on the surface of an anodic oxide film 2 having pores 3 that have a deposit 4 including an anti-virus inorganic compound deposited therein to be filled with the deposit 4, the anodic oxide film being similar to that of the first embodiment. The surface film 30 includes an inorganic fine particle 5 composed of an anti-virus inorganic compound, a functional fine particle 7 for imparting a function other than an anti-virus property, and a binder 8 composed of a silane compound. In certain usage environments, for example, a known hard coating agent may be added to improve the strength of the surface film 30 further.

An inorganic oxide can be used as the functional fine particle 7 used in the third embodiment of the present invention. Examples of the inorganic oxide may include a single inorganic oxide such as SiO₂, Al₂O₃, TiO₂, ZrO₂, SnO₂, Fe₂O₃, Sb₂O₃, WO₃, and CeO₂. A composite oxide may also be used. Examples of the composite oxide may include SiO₂·Al₂O₃, SiO₂·B₂O₃, SiO₂·P₂O₅, SiO₂·TiO₂, SiO₂·ZrO₂, Al₂O₃·TiO₂, Al₂O₃·ZrO₂, Al₂O₃·CaO, Al₂O₃·B₂O₃, Al₂O₃·P₂O₅, Al₂O₃·CeO₂, Al₂O₃·Fe₂O₃, TiO₂·CeO₂, TiO₂·ZrO₂, SiO₂·TiO₂·ZrO₂, Al₂O₃·TiO₂·ZrO₂, SiO₂·Al₂O₃·TiO₂, and SiO₁·TiO₂·CeO₂. Functional fine particles 7 with an average particle diameter of approximately 1 nm to 5 µm are used. When the functional fine particles are used, they are mixed into the surface film 30 in an amount of approximately 1% by mass to 80% by mass. Use of such an inorganic oxide improves the film strength of the surface film 30, thereby enhancing its abrasion resistance. As a result, a member that can exert an anti-virus effect stably for a long period of time can be provided.

A photocatalytic substance may also be used as the functional fine particle 7. A photocatalytic substance is a particle that performs a photocatalytic function when the substance is irradiated with light of a wavelength having energy exceeding the band gap of the substance. Examples of the photocatalytic substance may include a known metallic compound semiconductor, such as titanium oxide, zinc oxide, tungsten oxide, iron oxide, strontium titanate, cadmium sulfide, and cadmium selenide. These may be used alone or in a combination of two or more thereof.

Among these photocatalytic substances, titanium oxide, zinc oxide, and tungsten oxide are particularly preferable as the functional fine particle 7 used in the third embodiment of the present invention, because they are low in toxicity and excellent in safety. In the present invention, the crystal structure of titanium oxide, which is a photocatalytic substance, may be any of a rutile-type, an anatase-type, a brookite-type, and other types, and titanium oxide may be even amorphous.

Furthermore, a photocatalytic substance that has photocatalytic activity even under visible light, and the like may be used. Examples of such a photocatalytic substance may include TiO_{2-X}N_{X} in which part of the oxygen atoms of titanium oxide are substituted with a nitrogen atom which is an anion, TiO_{2-X} (X is 1.0 or less) that has lost an oxygen atom and deviates significantly from the stoichiometric ratio, titanium oxide supporting a nanoparticle of a copper compound or an iron compound, tungsten oxide supporting a nanoparticle of gold or silver, tungsten oxide doped with an iron ion or a copper ion, and zinc oxide doped with gold, iron, or potassium.

Furthermore, a metal such as vanadium, copper, nickel, cobalt, and chromium or a compound thereof, or a noble metal such as palladium, rhodium, ruthenium, silver, platinum, and gold or a metal compound thereof, or a monovalent copper compound such as CuCl, CuBr, Cu(CH₃COO), CuSCN, Cu₂S, Cu₂O, and CuI may be included inside or on the surface of these photocatalytic substances to enhance the photocatalytic function.

Furthermore, examples of the binder 8 composed of a silane compound used in the third embodiment of the present invention may include vinyltrichlorosilane, vinyltrimethoxysilane, vinyltriethoxysilane, vinyltriacetoxysilane, N-β-(N-vinylbenzylaminoethyl)-γ-aminopropyltrimethoxysilane, N-(vinylbenzyl)-2-aminoethyl-3-aminopropyltrimethoxysilane hydrochloride, 2-(3,4epoxycyclohexyl)ethyltrimethoxysilane, 3-glycidoxypropyltrimethoxysilane, 3-glycidoxypropylmethyldiethoxysilane, 3-glycidoxypropyltriethoxysilane, p-styryltrimethoxysilane, 3-methacryloxypropylmethyldimethoxysilane, 3-methacryloxypropyltrimethoxysilane, 3-methacryloxypropylmethyldiethoxysilane, 3-methacryloxypropyltriethoxysilane, 3-acryloxypropyltrimethoxysilane, 3-isocyanatepropyltriethoxysilane, bis(triethoxysilylpropyl)tetrasulfide, 3-aminopropyltrimethoxysilane, 3-aminopropyltriethoxysilane, 3-triethoxysilyl-N-(1,3-dimethyl-butylidene)propylamine, N-phenyl-3-aminopropyltrimethoxysilane, N-2-(aminoethyl)-3-aminopropylmethyldimethoxysilane, N-2-(aminoethyl)-3-aminopropyltrimethoxysilane, N-2-(aminoethyl)-3-aminopropyltriethoxysilane, 3-mercaptopropylmethyldimethoxysilane, 3-mercaptopropyltrimethoxysilane, N-phenyl-3-aminopropyltrimethoxysilane, special aminosilane, 3-ureidopropyltriethoxysilane, 3-chloropropyltrimethoxysilane, tetramethoxysilane, tetraethoxysilane, methyltrimethoxysilane, methyltriethoxysilane, dimethyldiethoxysilane, phenyltriethoxysilane, hexamethyldisilazane, hexyltrimethoxysilane, decyltrimethoxysilane, hydrolyzable group-containing siloxane, a fluoroalkyl group-containing oligomer, methyl hydrogen siloxane, and a silicon quaternary ammonium salt.

Furthermore, examples of the silane oligomer may include commercially available KC-89S, KR-500, X-40-9225, KR-217, KR-9218, KR-213, KR-510, and the like from Shin-Etsu Chemical Co. , Ltd. These silane oligomers are used alone or in a mixture of two or more thereof, and moreover, these may be used in a mixture with one or two or more of the binders 8 composed of a silane compound. When these binders 8 composed of a silane compound are used, they are mixed into the surface film 30 in an amount of approximately 1 to 50% by mass.

A method for producing the aluminum member 300 of this embodiment will be described below. First, the anodic oxide film 2 that has a large number of pores 3 formed therein is formed on the surface of aluminum or an aluminum alloy and the deposit 4 including an anti-virus inorganic compound is deposited within the pores 3 by the method described in the first embodiment. Next, the inorganic fine particles 5 composed of the anti-virus inorganic compound are pulverized, for example, by a jet mill or a hammer mill into nano-order particles, submicron-order particles, or micron-order particles. The pulverization process is not particularly limited and both a dry process and a wet process can be used. The inorganic fine particles 5 composed of the pulverized anti-virus inorganic compound are dispersed in a solvent such as water, methanol, ethanol, or toluene along with functional fine particles 7 that are composed of inorganic fine particles selected based on a required function, and they are pulverized again, for example, by a jet mill or a hammer mill. The slurry thus obtained is applied to the surface of the aluminum member 300 by a known method such as a dipping method, a spray method, or a screen printing method, and the solvent is removed if required, for example, by heating and drying. Subsequently, the binder 8 composed of a silane compound, a known hard coating agent, and the like are chemically bound to the surface of the aluminum member 300, for example, by graft polymerization by reheating or by graft polymerization by exposure to radiation, e.g., infrared rays, ultraviolet rays, an electron beam, and gamma rays.

According to the third embodiment described above, inorganic fine particles are chemically bound to each other on the surface of the anodic oxide film 2 through the binder 8 composed of a silane compound or a known hard coating agent, thereby forming a three-dimensional bridged structure. Therefore, an anti-virus component such as a monovalent copper ion that is released from the deposit 4 deposited within the pores 3 passes through microscopic gaps of this bridged structure and appears on the surface. Consequently, both anti-virus substances, that is, the anti-virus inorganic fine particles 5 on the surface film 30 and the deposit 4, can act on a virus. Thus, an aluminum member with a higher virus-inactivating ability can be provided. Furthermore, a functional fine particle that is selected from various inorganic compounds can be used to achieve an effect other than an anti-virus property. For example, the functional fine particle can improve the strength of the surface film 30 or impartaphotocatalytic function to the aluminum member. However, there is no need to add the functional fine particle 7 included in the surface film 30, for example, when the anti-virus aluminum member 300 of the present invention is used in an environment where a film strength or corrosion resistance is not needed.

### (Fourth Embodiment)

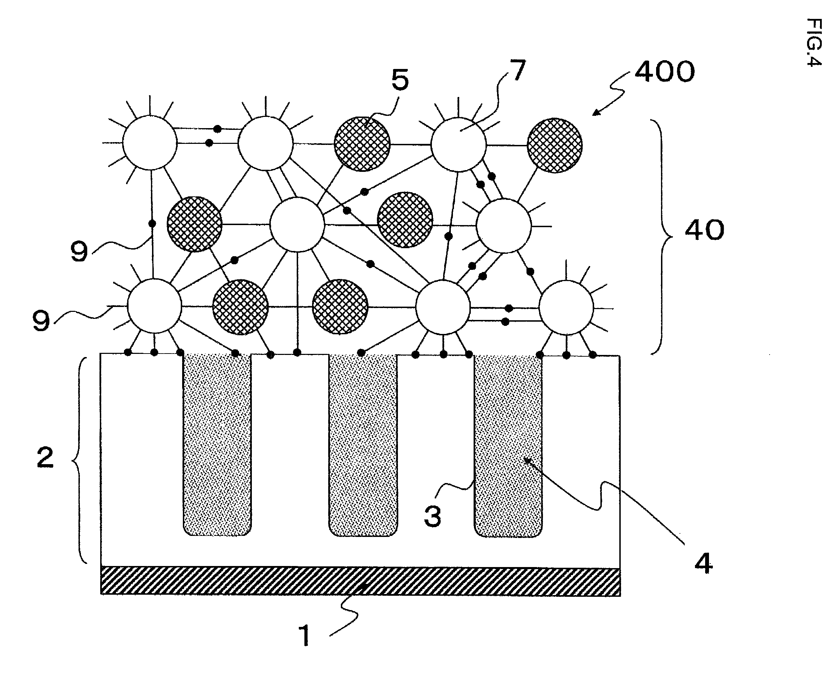

Next, an anti-virus aluminum member 400 of the fourth embodiment of the present invention will be described in detail with reference to Fig. 4.

Fig. 4 is an enlarged schematic view of part of the cross section of the anti-virus aluminum member 400 of the fourth embodiment of the present invention. In the fourth embodiment, a surface film 40 is formed on the surface of a porous anodic oxide film 2 that is filled with a deposit 4 including an anti-virus inorganic compound, the porous anodic oxide film 2 being similar to that of the first embodiment. The surface film 40 includes an anti-virus inorganic fine particle 5 composed of an inorganic compound and a functional fine particle 7 covered with a silane monomer 9 having a functional group capable of chemical bonding.

The silane monomer 9 having a functional group capable of chemical bonding that is used in the anti-virus aluminum member 400 of the fourth embodiment of the present invention is, for example, a silane monomer represented by a general formula X-Si(OR)ₙ(n is an integer of 1 to 3). For example, X is a functional group that reacts with an organic compound, such as a vinyl group, an epoxy group, a styryl group, a methacrylo group, an acryloxy group, an isocyanate group, a polysulfide group, an amino group, a mercapto group, or a chloro group. OR is a hydrolyzable alkoxy group such as a methoxy group and an ethoxy group and the three functional groups of the silane monomer 9 may be identical or different from each other. These alkoxy groups such as a methoxy group and an ethoxy group are hydrolyzed to produce a silanol group. The silanol group, a vinyl group, an epoxy group, a styryl group, a methacrylo group, an acryloxy group, an isocyanate group, and also a functional group having an unsaturated bond, and the like are known to be highly reactive. Thus, in the anti-virus aluminum member 400 of the fourth embodiment of the present invention, the inorganic fine particles 7 chemically bind to each other through such a silane monomer 9 excellent in reactivity, thereby forming a matrix. At the same time, the inorganic fine particles 7 also bind firmly to the anodic oxide film 2 having the pores 3. In this manner, the anti-virus aluminum member 400 that is excellent in strength can be provided.

A method for producing the anti-virus aluminum member 400 of this embodiment will be described below. First, the anodic oxide film 2 that has a large number of pores 3 formed therein is formed on the surface of aluminum or an aluminum alloy and the deposit 4 including an anti-virus inorganic compound is deposited within the pores 3 by the method described in the first embodiment. Next, the above-mentioned silane monomer 9 having a functional group capable of chemical bonding is added to a dispersion prepared by dispersing the functional fine particles 7 in a solvent. The silane monomer 9 is allowed to chemically bind to the surface of the functional fine particles 7 by a dehydration condensation reaction while heating at reflux. In this case, the amount of the silane monomer 9 may be 0.01% by mass to 40.0% by mass relative to the mass of the functional fine particles 7, although the amount varies depending on the average particle diameter of the functional fine particles 7. Then, the functional fine particles 7 thus obtained having their surfaces covered with the silane monomers and anti-virus inorganic fine particles 5 composed of a pulverized inorganic compound by the method described in the third embodiment are dispersed in a solvent. Then, the resulting dispersion is further pulverized, for example, by a jet mill or a hammer mill to obtain a slurry. The slurry thus obtained is applied onto the surface of the aluminum member 400 by a known method such as a dipping method, a spray method, or a screen printing method, and the solvent is removed if required, for example, by heating and drying. Subsequently, the functional group capable of chemical bonding of the silane monomer 9 is chemically bound to the surface of the aluminum member 400 (anodic oxide film 2), for example, by graft polymerization by reheating or by graft polymerization by exposure to radiation, e.g., infrared rays, ultraviolet rays, an electron beam, and gamma rays (radiation graft polymerization).

According to the fourth embodiment described above, the anti-virus inorganic fine particles 5 composed of the inorganic compound are held in the state where they are caught in the mesh of the three-dimensional bridged structure formed by chemical bonding among the silane monomers 9 bonded to the surface of the functional fine particles 7. Therefore, the surfaces of the inorganic fine particles 5 are not covered with the binders or the like. For this reason, almost the entire inorganic fine particle 5 can come into contact with a virus and the probability of contact with viruses increases, and therefore, even a small amount of inorganic fine particles 5 can inactivate viruses efficiently.

The anti-virus aluminum members according to the first to fourth embodiments described above can inactivate various viruses regardless of the type of their genomes or whether a viral envelope is present. Examples of such viruses may include a rhinovirus, a poliovirus, a foot-and-mouth disease virus, a rotavirus, a norovirus, an enterovirus, a hepatovirus, an astrovirus, a sapovirus, a hepatitis E virus, an influenza A virus, an influenza B virus, an influenza C virus, a parainfluenza virus, a mumps virus (mumps), a measles virus, a human metapneumovirus, an RS virus, a Nipah virus, a Hendra virus, a yellow fever virus, a dengue virus, a Japanese encephalitis virus, an West Nile virus, a hepatitis B virus, a hepatitis C virus, an eastern equine encephalitis virus and an western equine encephalitis virus, an O'nyong' nyong virus, a rubella virus, a Lassa virus, a Junin virus, a Machupo virus, a Guanarito virus, a Sabia virus, a Crimean-Congo hemorrhagic fever virus, a sandfly fever, a hantavirus, a Sin Nombre virus, a rabies virus, an Ebola virus, a Marburg virus, a lyssavirus, a human T cell leukemia virus, a human immunodeficiency virus, a human coronavirus, a SARS coronavirus, a human parvovirus, a polyoma virus, a human papillomavirus, an adenovirus, a herpesvirus, a varicella-zonal rash virus, an EB virus, a cytomegalovirus, a smallpox virus, a monkeypox virus, a cowpox virus, a molluscipoxvirus, and a parapoxvirus.

The anti-virus aluminum member obtained as described above can be used in a film (foil) shape, a plate shape, a linear shape, a tubular shape, and various other shapes. Specifically, the anti-virus aluminum member is applicable to various fields and can be used for a door knob, a handrail, a front door, a sash such as a window frame , a filter for an air-conditioner, a filter for an air cleaner, a filter for a cleaner, a filter for an extractor fan, a filter for a vehicle, a filter for air-conditioning equipment, a net for a screen door, a net for a henhouse, a fin material for an air-conditioner, a wall material or a ceiling material for an operating room or a bathroom, a wheelchair, a bed component, a safety cabinet for a virus test, and the like.

The present invention will now be described more specifically by way of Examples. However, the present invention is not limited only to these Examples.

### [Examples]

### (Production of anti-virus aluminum member)

### (Example 1)

First, an aluminum plate material (JISH1050 material) was immersed for 60 seconds in 5% sodium hydroxide aqueous solution heated to 50°C as pretreatment, and then, alkali was neutralized and removed by immersing the aluminum plate material in 5% nitric acid aqueous solution. Next, anodization at a current density of 1.5 A/dm² for 20 minutes was carried out in an electrolyte at a temperature of 20°C containing 1.5 mol of sulfuric acid, with the pretreated aluminum plate material serving as an anode and a platinum electrode serving as a counter electrode (cathode). By this anodization, a porous anodic oxide film approximately 8 µm in thickness was formed on the surface of the aluminum plate material.

Then, the aluminum plate material on which the porous anodic oxide film approximately 8 µm in thickness was formed was immersed in an aqueous solution containing 40 g/L copper sulfate and 10 g/L boric acid, and an alternating current voltage of 10 V was applied, with a platinum electrode serving as a counter electrode. In this manner, a deposit including a monovalent copper compound was deposited within the pores of the anodic oxide film, thereby producing an anti-virus aluminum member. In Example 1, three types of aluminum members were produced by adopting a treatment time (voltage application time) of 1 minute, 5 minutes, and 10 minutes. The example with a treatment time of 1 minute is referred to as Example 1-1, the example with a treatment time of 5 minutes is referred to as Example 1-2, and the example with a treatment time of 10 minutes is referred to as Example 1-3.

### (Example 2)

In Example 2, a resin containing anti-virus inorganic fine particles was applied onto the surface of the aluminum member of Example 1. First, copper (I) iodide powder (manufactured by Nihon Kagaku Sangyo Co., Ltd.) was pulverized into fine particles with an average particle diameter of 140 nm by a dry pulverizer, Nano Jetmizer (manufactured by Aishin Nano Technologies CO., LTD., NJ-100B), to produce anti-virus inorganic fine particles. The obtained fine particles were added to a two-component silicon acrylic resin coating (manufactured by Natoco Co., Ltd., Arco SP) so that the contained amount of the fine particles in the coating film after drying was 5% by mass, and the fine particles were dispersed using a ball mill. Octadecylamine acetate (manufactured by NOF CORPORATION., Nissan cation SA) was also added as a surface active agent in an amount of 0.2% by mass relative to the solid content of the coating. Then, onto the surface of the aluminumplate produced in Example 1-3 that had a deposit including a monovalent copper compound deposited within the pores of the anodic oxide film under a condition in which the treatment time was 10 minutes, the above-mentioned silicon acrylic resin coating was applied by spraying. The coating included the copper (I) iodide fine particles and the surface active agent dispersed therein. The aluminum plate was dried for 20 minutes at 160°C to produce the anti-virus aluminum plate of Example 2.

### (Example 3)

An anti-virus aluminum plate of Example 3 was produced by a similar method and under a similar condition to those of Example 2, except that silver iodide powder (manufactured by Wako Pure Chemical Industries, Ltd.) was used instead of copper iodide powder, which was used for the anti-virus inorganic fine particles in Example 2. The silver iodide powder was pulverized into fine particles with an average particle diameter of 800 nm by a dry pulverizer, Nano Jetmizer (manufactured by Aishin Nano Technologies CO., LTD., NJ-1003).

### (Example 4)

In Example 4, anti-virus inorganic fine particles and photocatalytic fine particles serving as functional fine particles were immobilized on the surface of the aluminum member of Example 1. The copper iodide powder used in Example 2 and fine particles of iron ion-doped anatase titanium oxide, which is a visible light-responsive photocatalytic substance, (manufactured by Ishihara Sangyo Kaisha, Ltd., MPT-625) were predispersed in methanol. Subsequently, the dispersion was pulverized and dispersed by a bead mill to obtain a slurry including both the fine particles of copper (I) iodide with an average particle diameter of 45 nm and the fine particles of iron ion-doped anatase titanium oxide, which is a visible light-responsive photocatalytic substance, with an average particle diameter of 82 nm. Tetramethoxysilane (manufactured by Shin-Etsu Chemical Co., Ltd., KBM-04) was added as a binder in an amount of 40% by mass relative to the solid content of the obtained slurry, and methanol was added to adjust the concentration of the solid content to 5% by mass. The amount of the fine particles of copper (I) iodide to be added was adjusted so that the amount of copper (I) iodide that remained after the solvent was removed by drying the slurry on the substrate surface (on the anodic oxide film) was 1.0% by mass relative to the solid content on the substrate. The solid content represents the total amount of the fine particles of copper (I) iodide and the fine particles of iron ion-doped anatase titanium oxide, which is a visible light-responsive photocatalytic substance.

Then, onto the surface of the aluminumplate produced in Example 1-3 that had a deposit including a monovalent copper compound deposited within the pores of the anodic oxide film under a condition in which the treatment time was 10 minutes, the above-mentioned slurry was applied by spraying. The slurry included the fine particles of copper (I) iodide, the fine particles of titanium oxide, and tetramethoxysilane and was adjusted by adding methanol. The aluminum plate was dried for 20 minutes at 180°C to produce the anti-virus aluminum plate of Example 4.

### (Example 5)

In Example 5, anti-virus inorganic fine particles and functional fine particles covered with silane monomers were immobilized on the surface of the anti-virus aluminum member of Example 1. First, the copper iodide powder used in Example 2 and zirconium oxide particles (manufactured by Nippon Denko Co., Ltd., PCS) were predispersed in methanol. The zirconium oxide particle has methacryloxypropyltrimethoxysilane (manufactured by Shin-Etsu Chemical Co., Ltd., KBM-503) which is a silane monomer having an unsaturated bond part. The methacryloxypropyltrimethoxysilane is covalently bonded to the surface of the zirconium oxide particle by a dehydration-condensation by ordinary method. Subsequently, the dispersion was pulverized and dispersed by a bead mill to obtain a slurry including particles of copper (I) iodide with an average particle diameter of 45 nm and particles of zirconium oxide with an average particle diameter of 37 nm covered with methacryloxypropyltrimethoxysilane. Tetramethoxysilane (manufactured by Shin-Etsu Chemical Co., Ltd., KBM-04) was added as a binder in an amount of 20% by mass relative to the solid content of the obtained slurry, and methanol was added to adjust the concentration of the solid content to 5% by mass. The amount of the fine particles of copper (I) iodide to be added was adjusted so that the amount of copper (I) iodide that remained after the solvent was removed by drying the slurry on the substrate surface (on the anodic oxide film) is 1.0% by mass relative to the solid content on the substrate. The solid content represents the total amount of the fine particles of copper (I) iodide and the fine particles of zirconium oxide with methacryloxypropyltrimethoxysilane bound thereto.

Then, onto the surface of the aluminumpla te produced in Example 1-3 that had a deposit including a monovalent copper compound deposited within the pores of the anodic oxide film under a condition in which the treatment time was 10 minutes, the above-mentioned slurry was applied by spraying. The slurry included the fine particles of copper (I) iodide, the particles of zirconium oxide, and tetramethoxysilane and was adjusted by adding methanol. The aluminum plate was dried for 20 minutes at 180°C to produce the anti-virus aluminum plate of Example 5.

### (Example 6)

An anti-virus aluminum plate of Example 6 was produced by a similar method and under a similar condition to those of Example 5, except that 30% by mass of the fine particles of zirconium oxide of Example 5 with methacryloxypropyltrimethoxysilane bound thereto were replaced by fine particles of anatase titanium oxide (manufactured by Tayca Corporation, AMT-100) with methacryloxypropyltrimethoxysilane bound thereto. The anatase titanium oxide is a photocatalytic substance.

### (Example 7)

An anti-virus aluminum plate of Example 7 was produced by a similar method and under a similar condition to those of Example 5, except that 30% by mass of the fine particles of zirconium oxide of Example 5 with methacryloxypropyltrimethoxysilane bound thereto were replaced by fine particles of iron ion-doped anatase titanium oxide (manufactured by Ishihara Sangyo Kaisha, Ltd. , MPT-625). The iron ion-doped anatase titanium oxide is a visible light-responsive photocatalytic substance.

### (Example 8)

An anti-virus aluminum plate of Example 8 was produced by a similar method and under a similar condition to those of Example 5, except that commercially available silver iodide (manufactured by Wako Pure Chemical Industries, Ltd.) was used instead of the copper iodide powder used in Example 5.

### (Example 9)

In Example 9, an anodic oxide film having pores was formed on the surface of an aluminum plate material under a similar condition to that of Example 1. Subsequently, an alternating current voltage of 10 V was applied in an aqueous solution containing copper sulfate for 2 minutes under a similar condition to that of Example 1. Then, the aluminum plate material was immersed in an aqueous solution containing 0. 05 mol/L potassium iodide and a direct current voltage was applied at a current density of 0.1 A/dm² for 3 minutes, with a platinum electrode serving as a counter electrode. In this manner, a deposit including copper (I) iodide was synthesized and deposited within the pores of the anodic oxide film, thereby producing an anti-virus aluminum plate.

### (Example 10)

In Example 10, an anodic oxide film having pores was formed on the surface of an aluminum plate material under a similar condition to that of Example 1. Subsequently, the aluminum plate material was immersed in an aqueous solution containing 5 g/L silver nitrate, and an alternating current voltage of 8 V was applied for 10 minutes, with a platinum electrode serving as a counter electrode. Consequently, a deposit including silver was deposited within the pores of the anodic oxide film. Then, the aluminum plate material having the deposit including silver filling the pores of the anodic oxide film was immersed in an aqueous solution containing 0.05 mol/L potassium iodide and a direct current voltage was applied at a current density of 0.17 A/dm² for 3 minutes, with a platinum electrode serving as a counter electrode. In this manner, a deposit including silver iodide was synthesized and deposited within the pores of the anodic oxide film, thereby producing an anti-virus aluminum plate.

### (Example 11)

In Example 11, an anodic oxide film having pores was formed on the surface of an aluminum plate material under a similar condition to that of Example 1. Subsequently, a current density of 0.1 A/dm² was applied for 10 minutes, with a platinum electrode serving as a counter electrode, in an aqueous solution containing silver iodide with an average particle diameter of 2 nm, which was prepared by mixing silver nitrate and potassium iodide. In this manner, a deposit including silver iodide was deposited within the pores of the anodic oxide film, thereby producing an anti-virus aluminum plate.

### (Comparative Example 1)

The aluminum plate having an anodic oxide film formed thereon produced in Example 1 (the one that was not subjected to the process for depositing a copper compound within its pores) was used as Comparative Example 1.

### (Comparative Example 2)

Commercially available pure copper plate (JISH3100 material manufactured by U-KOU Co. Ltd.) was immersed in methanol for 1 minute at room temperature to remove a film formed by natural oxidation on the surface of the copper plate. Then, the plate was dried at room temperature and used as Comparative Example 2.

The compositions of Examples 1 to 11 and Comparative Examples 1 and 2 are shown in Table 1.

**[Table 1]**

| | METAL PLATE MATERIAL | SUBSTANCES WITHIN PORES (DEPOSIT PROCESS TIME) | COATING ON ANODIC OXIDE FILM |
|---|---|---|---|
| Example 1-1 | Al + ANODIC OXIDE FILM | MONOVALENT COPPER COMPOUND (1 min) | NONE |
| Example 1-2 | Al + ANODIC OXIDE FILM | MONOVALENT COPPER COMPOUND (5 min) | NONE |
| Example 1-3 | Al + ANODIC OXIDE FILM | MONOVALENT COPPER COMPOUND (10 min) | NONE |
| Example 2 | Al + ANODIC OXIDE FILM | MONOVALENT COPPER COMPOUND (10 min) | COPPER (I) IODIDE + RESIN + SURFACE ACTIVE AGENT |
| Example 3 | Al + ANODIC OXIDE FILM | MONOVALENT COPPER COMPOUND (10 min) | SILVER IODIDE + RESIN + SURFACE ACTIVE AGENT |
| Example 4 | Al + ANODIC OXIDE FILM | MONOVALENT COPPER COMPOUND (10 min) | COPPER (I) IODIDE + IRON ION-DOPED TITANIUM OXIDE + TETRAMETHOXYSILANE (BINDER) |
| Example 5 | Al + ANODIC OXIDE FILM | MONOVALENT COPPER COMPOUND (10 min) | COPPER (I) IODIDE + ZIRCONIUM OXIDE COVERED WITH SILANE MONOMER + TETRAMETHOXYSILANE (BINDER) |
| Example 6 | Al + ANODIC OXIDE FILM | MONOVALENT COPPER COMPOUND (10 min) | COPPER (I) IODIDE + ZIRCONIUM OXIDE COVERED WITH SILANE MONOMER + TITANIUM OXIDE COVERED WITH SILANE MONOMER + TETRAMETHOXYSILANE (BINDER) |
| Example 7 | Al + ANODIC OXIDE FILM | MONOVALENT COPPER COMPOUND (10 min) | COPPER (I) IODIDE + ZIRCONIUM OXIDE COVERED WITH SILANE MONOMER + IRON ION-DOPED TITANIUM OXIDE COVERED WITH SILANE MONOMER + TETRAMETHOXYSILANE (BINDER) |
| Example 8 | Al + ANODIC OXIDE FILM | MONOVALENT COPPER COMPOUND (10 min) | SILVER IODIDE + ZIRCONIUM OXIDE COVERED WITH SILANE MONOMER + TETRAMETHOXYSILANE (BINDER) |
| Example 9 | Al + ANODIC OXIDE FILM | MONOVALENT COPPER COMPOUND INCLUDING CuI | NONE |
| Example 10 | Al + ANODIC OXIDE FILM | MONOVALENT COPPER COMPOUND INCLUDING AgI | NONE |
| Example 11 | Al + ANODIC OXIDE FILM | MONOVALENT COPPER COMPOUND INCLUDING AgI | NONE |
| Comparative Example 1 | Al + ANODIC OXIDE FILM | NONE | NONE |
| Comparative Example 2 | COPPER | NONE | NONE |

### (Analysis of anodic oxidation film on aluminum by wide-angle X-ray diffraction)

Substances at approximately 6 µm depth below the surface of the anti-virus aluminum plates of Example 1, Example 9, and Example 10 were analyzed by a wide-angle X-ray diffractometer (manufactured by Rigaku Corporation). In the case of the anti-virus aluminum plate obtained in Example 1, a diffraction pattern was obtained that included a peak at 2θ = 36.5° associated with the (111) plane of Cu₂O, a peak at 2θ = 42.4° associated with the (200) plane of Cu₂O, and a peak at 2θ = 61.6° associated with the (220) plane of Cu₂O. In Example 9, a diffraction pattern was obtained that included a peak at 2θ = 25.3° associated with the (111) plane of CuI, a peak at 2θ = 41.8° associated with the (220) plane of CuI, and a peak at 2θ = 49.5° associated with the (311) plane of CuI. In Example 10, a diffraction pattern was obtained that included a peak at 2θ = 22.3° associated with the (100) plane of AgI, a peak at 2θ = 25.3° associated with the (101) plane of AgI, and a peak at 2θ = 42.6° associated with the (103) plane of AgI. These results confirmed that a monovalent copper compound or an iodine compound was deposited within the pores of respective anodic oxide films.

### (Evaluation of virus inactivation)

Measurement of the virus-inactivating ability of an anti-virus aluminum member was performed by using an influenza virus A/Kitakyushu/159/93(H3N2) as an enveloped virus and a feline calicivirus (strain F9), which is generally used as an alternative to a norovirus, as a nonenveloped virus. As for these used viruses, the influenza virus (influenza A/Kitakyushu/159/93(H3N2)) was cultivated by using MDCK cells and the feline calicivirus (strain F9) was cultivated by using CRFK cells. A 4 cm x 4 cm sample of each of Examples and Comparative Examples was placed in a plastic petri dish, and 0.1 mL of a virus solution was dropped onto the sample and was allowed to act for 30 minutes at room temperature. At this time, the contact area of the virus solution and the sample was kept constant by covering the surface of the sample with a PET film (4 cm x 4 cm). After allowing the virus solution act for 30 minutes, 1900 µl of SCDLP broth was added and the viruses were washed out by pipetting. Then, each of the reaction samples were diluted with an MEM broth to make 10⁻² to 10⁻⁵ dilutions (10-fold serial dilution). One hundred microliters of the sample solution was inoculated into the MDCK cells or the CRFK cells that had been cultivated in a petri dish. After allowing the culture to stand for 60 minutes and the viruses to be adsorbed by the cells, a 0.7% agar medium was overlaid on the culture in the petri dish. After cultivation at 34°C for 48 hours in a 5% CO₂ incubator, the culture was fixed in formalin. The number of plaques formed by methylene blue staining was counted and the viral infectivity titer (PFU/0.1 mL, Log 10) (PFU: plaque-forming units) was calculated. The value obtained when only the virus solution was added and the samples of Examples were not used was used as a control. The results are shown in Table 2.

**[Table 2]**

| | VIRAL INFECTIVITY TITER (PFU/0.1ml, Log10) | |
|---|---|---|
| | INFLUENZA VIRUS | FELINE CALICIVIRUS |
| | TYPE A (H3N2) | STRAIN F9 |
| Example 1-1 | <1.3 | <1.3 |
| Example 1-2 | 1.8 | <1.3 |
| Example 1-3 | <1.3 | <1.3 |
| Example 2 | <1.3 | <1.3 |
| Example 3 | 3.7 | 3.5 |
| Example 4 | <1.3 | <1.3 |
| Example 5 | <1.3 | <1.3 |
| Example 6 | <1.3 | <1.3 |
| Example 7 | <1.3 | <1.3 |
| Example 8 | 3.5 | 3.2 |
| Example 9 | 4.2 | 4.0 |
| Example 10 | 5.1 | 4.9 |
| Example 11 | 5.2 | 5.0 |
| Comparative Example 1 | 6.2 | 6.2 |
| Comparative Example 2 | 6.1 | 6.2 |
| CONTROL | 6.8 | 7.0 |

The above results confirmed that the infectivity titers were reduced in all of Examples 1 to 11 regardless of whether a viral envelope is present. In particular, Examples 1 and 2 and Examples 4 to 7 showed a very effective inactivation rate of 99.999% or more, after 30 minutes of exposure to viruses.

### Reference Signs List

- 1: Metal layer
- 2: Anodic oxide film
- 3: Pore
- 4: Deposit
- 5: Inorganic fine particle
- 6: Resin binder
- 7: Functional fine particle
- 8: Binder (silane compound)
- 9: Silane monomer
- 10, 30, 40: Surface film
- 100, 200, 300, 400: Aluminum member

## Claims

1. An anti-virus aluminum member that can inactivate a virus adhering to the anti-virus aluminum member, wherein
an anodic oxide film obtained by anodizing aluminum or an aluminum alloy has a large number of pores, and an anti-virus inorganic compound is present within the pores.

2. The anti-virus aluminum member according to claim 1, wherein a surface film is formed on a surface of the anodic oxide film that has the anti-virus inorganic compound present within the pores, the surface film including an anti-virus inorganic compound and a binder resin.

3. The anti-virus aluminum member according to claim 2, wherein the surface film further includes an inorganic fine particle different from the anti-virus inorganic compound.

4. The anti-virus aluminum member according to claim 3, wherein the inorganic fine particles included in the surface film are a photocatalytic substance.

5. The anti-virus aluminum member according to claim 4, wherein the photocatalytic substance is a visible light-responsive photocatalytic substance.

6. The anti-virus aluminum member according to any one of claims 3 to 5, wherein a surface of the inorganic fine particle included in the surface film is covered with a silane monomer.

7. The anti-virus aluminum member according to any one of claims 2 to 6, wherein the binder resin is a silane compound.

8. The anti-virus aluminum member according to any one of claims 1 to 7, wherein the anti-virus inorganic compound is at least one of a monovalent copper compound and an iodine compound.

9. The anti-virus aluminum member according to claim 8, wherein the monovalent copper compound is at least one of a chloride, an acetic acid compound, a sulfide, an iodine compound, a bromide, a peroxide, an oxide, and a thiocyanide.

10. The anti-virus aluminum member according to claim 9, wherein the monovalent copper compound is at least one of CuCl, CuBr, Cu(CH₃COO), CuSCN, Cu₂S, Cu₂O, and CuI.

11. The anti-virus aluminum member according to any one of claims 8 to 10, wherein the iodine compound is at least one of CuI, AgI, SbI₃, IrI₄, GeI₄, GeI₂, SnI₂, SnI₄, TlI, PtI₂, PtI₄, PdI₂, BiI₃, AuI, AuI₃, FeI₂, CoI₂, NiI₂, ZnI₂, HgI, and InI₃.

12. A method for producing an anti-virus aluminum member, comprising the steps of:
anodizing an aluminum material made of aluminum or an aluminum alloy to form pores on a surface of the aluminum material; and
depositing an anti-virus inorganic compound, within the pores of the aluminum material with the pores formed on the surface of the aluminum material, by electrochemical treatment.

13. The method for producing an anti-virus aluminum member according to claim 12, wherein the step of depositing the anti-virus inorganic compound comprises:
depositing at least one of Cu and Ag within the pores by electrochemical treatment;
immersing the aluminum material with at least one of Cu and Ag having been deposited within the pores in an iodine ion-containing electrolyte; and
depositing CuI or AgI, which is the anti-virus inorganic compound, within the pores by electrochemical treatment of the immersed aluminum material.
